(19) Europäisches Patentamt
European Patent Office
Office européen des brevets

(11) Publication number: **0 076 973**
**B1**

(12) # EUROPEAN PATENT SPECIFICATION

(45) Date of publication of patent specification: **02.04.86**

(51) Int. Cl.$^4$: **C 07 D 407/04,** A 61 K 31/34

(21) Application number: **82109023.0**

(22) Date of filing: **29.09.82**

(54) **A new dioxolanic compound and its method of preparation; a new dioxolanic amine with pharmacological activity, its method of preparation, and the pharmaceutical compositions containing them.**

(30) Priority: **02.10.81 ES 505978**
**02.10.81 ES 505979**

(43) Date of publication of application:
**20.04.83 Bulletin 83/16**

(45) Publication of the grant of the patent:
**02.04.86 Bulletin 86/14**

(84) Designated Contracting States:
**BE DE FR GB IT**

(56) References cited:
**US-A-3 248 401**
**US-A-3 972 900**

**Melson et al, Acta Biol. Med., (Germ.), Band 6, 395 (1961)**

(73) Proprietor: **Investigacion Tecnica y Aplicada, S.A.**
**Poligono Industrial Santiga 6 calle Argenters**
**Santa Perpetua de Mogoda Barcelona (ES)**

(72) Inventor: **Ribalta, José Miguel B.**
**125 bis, calle Mayor de Sarriá**
**Barcelona (ES)**
Inventor: **Bruseghini, Leonida**
**5, calle Caponata**
**Barcelona (ES)**
Inventor: **Casadio, Silvano**
**11, via Tantardini**
**Mailand (IT)**
Inventor: **Iniesta, Jorge P.**
**39, calle Leyva**
**Barcelona (ES)**

(74) Representative: **Sandmair, Kurt, Dr. et al**
**Patentanwälte Schwabe, Sandmair, Marx**
**Stuntzstrasse 16**
**D-8000 München 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 076 973 B1

**Description**

This present invention refers to a new dioxolanic amine with pharmacological activity which has been allocated the reference ITA 362, for brevity.

This new compound has the following formula I

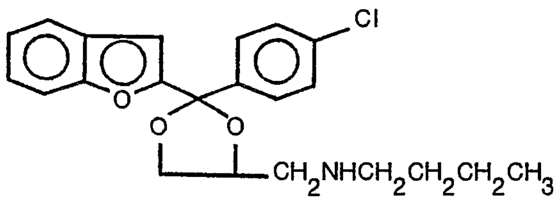

I

corresponding to the 2-(2'-benzofuryl)-4-n-butilaminomethyl-2-p-chlorophenyl-1, 3-dioxolane. Equally objects of the invention are the pharmaceutically acceptable salts of the said compound ITA 362, such as maleate, citrate, orotate, oxalate, malonate and p-toluenesulphonate.

The invention also refers to the method of preparation of the aforementioned compound ITA 362.

The new compound ITA 362 possesses intense vasodilatory, anti-angina and anti-arrhythmic activity, accompanied by a low toxicity. The use of the compound ITA 362 is another object of the invention.

The compounds of the invention comprises a combination of two chemical structures which possess good vasodilatory or anti-angina activity. One is the benzofurane structure described in US patent 3,248,401; the other structure is the dioxolane radical. Some compounds containing dioxolane groups are described by Melson et al, Acta Biol. Med. (Germ), Band 6, 395 (1961). A combination of both structures is not known in the art.

As will be explained in detail further on in the description of the chemical synthesis, in order to prepare the compound ITA 362 one starts off from the dioxolanic derivative bromomethyl of formula II of which the structure is the following;

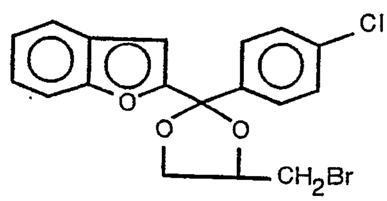

II

In fact this compound II is the 2-(2'-benzofuryl)-4-bromomethyl-2-p-chlorophenyl-1,3-dioxolane, which was obtained for the first time by the same inventors during the process of the research that led to the preparation of the compound ITA 362. Hence the said compound, of formula II is a new product as also following logically, is its method of preparation.

The invention refers also to this new product of formula II which is an intermediate compound in the preparation of the formula I compound. The other object of the invention is the method of preparation for the formula II compound.

*Chemical Synthesis*

The said compound ITA 362 as in formula I is synthesized chemically by means of a two stage process, these being;

a) first stage:

having for its object the preparation of formula II, that is to say; 2-(2'-benzofuryl)-4-bromomethyl-2-chlorophenyl-1,3-dioxolane.

b) second stage:

having for its object the preparation of the compound ITA 362 starting off from formula II.

Nevertheless, both stages, first and second, though having their individual identities, together form on unity of invention. The two said stages are described in detail further on.

First stage

The preparation of the bromomethyl dioxolanic derivative of the formula II, that is the 2-(2'-benzofuryl)-4-bromomethyl-2-p-chlorophenyl-1,3 dioxolane, takes place from starting with the Ketone of formula II

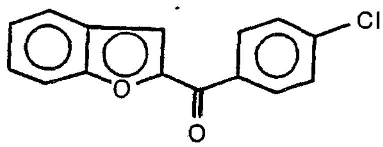

III

in a reaction of which the scheme is the following:

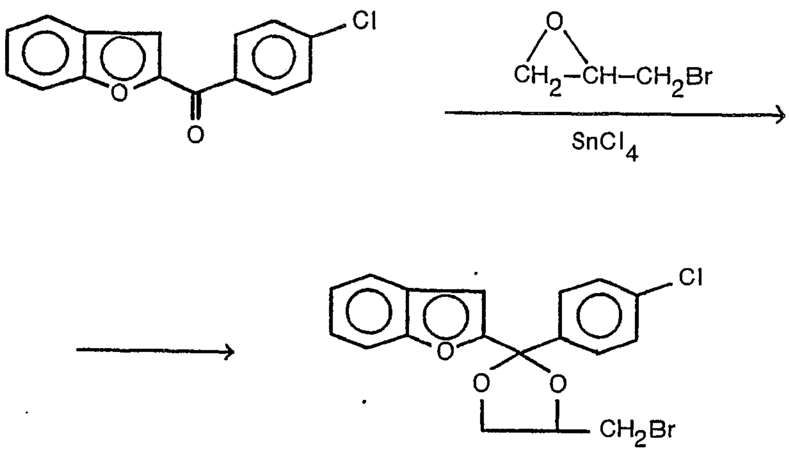


III

II

To a solution of Ketone III in a chloro solvent such as methylene chloride, chloroform or carbon tetrachloride is added at low temperature, (preferably around 0—5°C) a quantity of between 1 and 3 times mol. with respect to the Ketone III of epibromohydrin. Then adds slowly the catalyst corresponding to a Lewis acid (preferably tin tetrachloride, boron trifluoride or anhydrous aluminium trichloride) in a quantity of the order of one tenth part mol. with respect to the epibromohydrin, maintaining the mixture at a low temperature during the addition.

Then reaction is completed at a temperature of between the ambient and that of the boiling point of the mixture during a suitable time (from 2 to 24 hours).

Having completed the reaction a solution of sodium or potassium hydroxide is added quickly up to basic pH, then, the organic phase is decanted, washed, dried and the solvent removed. The residue thus obtained is distilled at a reduced pressure.

The structure of this new product corresponds to the 2-(2'-benzofuryl)-4-bromomethyl-2-p-chlorophenyl-1,3-dioxolane, which has two isomers due to the rigidity produced in the molecule by the dioxolane ring. These isomers are reflected by the nuclear magnetic resonance signal of the furan proton.

This type of isomer is described in great detail for systems similar to ours by P. Calinaud and J. Gelas (Bull.Soc. Chem. Fr. *5—6*, 1228—1236 and 1237—1242, 1975) and if we take as the principal substituent in the position of 2 of the dioxolan ring the benzofuran residue, the structures IV and V as shown in the following schemes, are formed:

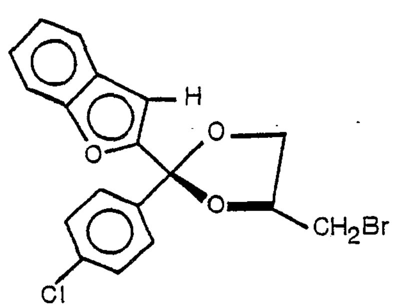

trans-
IV

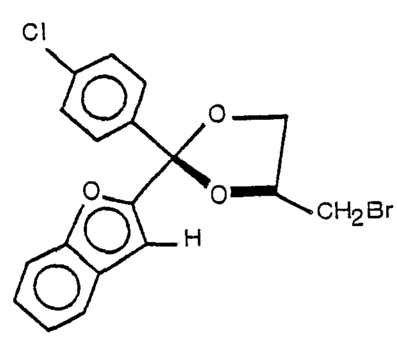

cis-
V

The product so obtained is used for the following or second stage.

Second Stage

The preparation of the ITA 362 is done starting from the 2-(2′-benzofuryl)-4-bromomethyl-2-p-chlorophenyl-1,3-dioxolane of formula II, as in the scheme hereunder:

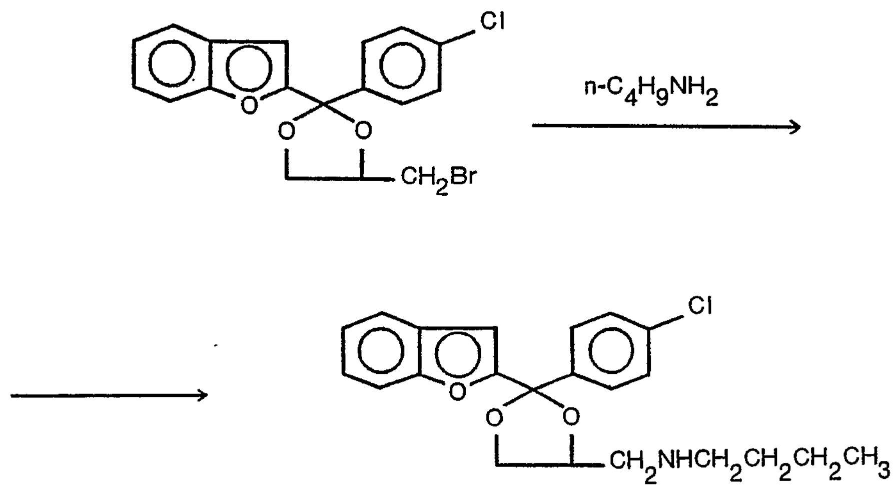


II

I

The 2-(2′-benzofuryl)-4-bromomethyl-2-p-chlorophenyl-1,3-dioxolane is caused to react with n-butylamine in an inert anhydrous solvent, preferably an aromatic hydrocarbon at the boiling point temperature of the mixture during the time required to complete the reaction (from 5 to 50 hours) and in the presence of an acid acceptor. As the acid acceptor the same n-butylamine may be used, using at least 2 mols of this for each mol of the bromomethyl derivative. Equally may be used as acid acceptor a tertiary amine such as triethylamine or pyridine, or even an inorganic base such as anhydrous sodium or potassium carbonate.

After the reaction is completed the mixture is allowed to cool and a solution of sodium or potassium hydroxide is added quickly, then, the organic phase is decanted, washed, dried, and the solvent removed yielding the product in the form of a reddish yellow oil which can be purified by distillation at a low pressure.

Treatment with an acid, preferably a weak organic acid such as maleic, citric, orotic, fumaric, malonic, acetic, p-toluenesulphonic or similar acid, in a suitable organic solvent such as acetone, methanol, ethanol, isopropanol, chloroform, ethyl acetate, benzene, and in general any organic solvent which would adequately dissolve the dioxolanic compound, allows to obtain a saline solid salt derivative which is more convenient for future handling and use. When the acid used has at the same time geometric or optical isomerism as is the case with maleic and fumaric acid, on the formation of the corresponding salts one can separate by fractional crystallization the two diastereoisomers which may be present, thus obtaining by this means the resolution of the isomers.

## Examples

Preliminary example (first stage)

We describe below an example of the preparation of the 2-(2′-benzofuryl)-4-bromomethyl-2-p-chlorophenyl-1,3-dioxolane.

To a solution of 136 g (0,53 m) of p-chlorobenzoyl-2-benzofurane in 2000 cc of anhydrous chloroform at 0—5 deg. C is added 109 g (0.80 m) of epibromohydrine. Then is added slowly (45 minutes) 9,4 cc (0,08 m) of tin tetrachloride, dissolved in 35 cc of anhydrous chloroform.

Having terminated the addition above, leave to attain room temperature and keep stirring during 15 hours. By means of TLC (eluent: benzene/hexane 4:1) the progress of the reaction can be controlled.

Quickly add 32 g of NaOH dissolved in 100 cc of water ensuring that the final pH is basic and stir during 30 minutes. Decant, wash the organic phase with water until the washings are neutral pH, dry the chloroform solution with $Na_2SO_4$ and elliminate the solvent at a low pressure obtaining 224 g of a yellowish oil.

Molecular distillation at 145 deg. C and approximately 1,3 $N/m^2$ ($10^{-2}$ mm Hq), yields 143 g (69%) of a slightly yellow oil. A further distillation of the residue of the previous operation yields a further 41 g. (total yield: 88%).

Halogens; (Schöninger): experimental = 28,84; theoretical: 29,30.

IR (film): Characteristic bands at 1595, 1490, 1450, 825 and 750 $cm^{-1}$.

NMR ($CDCl_3$): δ = 7,75—6,85 (m, 8, aromatics); 6,65 and 6,55 two singlets corresponding to the furan proton for the cis- and the trans-isomers; 4,80—3,20 (m, 5, aliphatics).

The experimental ratio of the aliphatic/aromatic integrals (theoretical: 0,555) is a very significant analytic measurement for testing the quality of the resulting product.

EM: Molecular peak at m/e 392 and 394 with an isotopic ratio suiting a bromine atom and a chlorine atom.

This product so obtained is ready for the aforementioned second stage leading to the preparation of the 2-(2'-benzofuryl)-4-n-butylaminomethyl-2-p-chlorophenyl-1,3-dioxolane. Should in any instance the reaction not be complete, most of the unreacted product may be elliminated by precipitation with cyclohexane and if necessary carrying out a previous distillation at 130 deg. C and 1,3 N/m$^2$ ($10^{-2}$ mm Hg) to remove the more volatile components.

Examples of the preparation procedure for obtaining the new dioxolanic amine of formula I (ITA 362) and its salts are described below, this being the second stage thereof.

Example 1

Preparation of the 2-(2'-benzofuryl)-4-n-butylaminomethyl-2-p-chlorophenyl-1,3-dioxolane. (Equally corresponds to the reference ITA 362)

A solution of 266 g (0.676 m) of 2-(2'-benzofuryl)-4-bromomethyl-2-p-chlorophenyl-1,3-dioxane and 400 cc (4,06 m) of n-butylamine in 1000 cc of anhydrous toluene is set to boil during 30h. By means of TLC (eluent AcOEt) the progress of the reaction can be controlled. Leave to cool and quickly add 300 cc of NaOH 2N and stir during 30 minutes. Decant, wash thoroughly the organic phase with water to remove the excess n-butylamine and eliminate the solvent at a low pressure, obtaining 255 g (98%) of a very thick reddish oil.

This product is of a quality suitable for the subsequent preparation of the salts. If required, further purification may be carried out by molecular distillation at 175 deg.C and 4—5 N/m$^2$ (3—4.$10^{-2}$ mm Hg) yielding 221 g (85%) of a very thick yellowish oil.

Potentiometric evaluation of basic groups: 103%.

IR (film): characteristic bands at 1600, 1450, 1250, 1090, 830 and 755 cm$^{-1}$.

NMR ($CDCl_3$): $\delta$ = 7,80—6,85 (m, 8, aromatics); 6,70 and 6,55 two singlets corresponding to the furan proton for the cis- and trans- isomers; 4,80—3,80 (m, 3, —O—$CH_2$—CH—O—); 3,00—2,45 (m, 4, —$\underline{CH_2}$NH—$\underline{CH_2}$); 1,80—0,70 (m, 7, —$\underline{CH_2CH_2CH_3}$).

The amine proton may appear as a singlet with $\delta$ approx. 2,2—2,5 or even included within the other signals in this zone in the form of a wide signal.

EM: molecular peak at m/e = 385.

The preparation of salts of the 2-(2'-benzofuranil)-4-n-butylaminemethyl-2-p-chlorphenyl-1,3-dioxolane. (ITA 362)

Because the ITA 362 base is an oil it is more convenient for its manipulation and usage to be converted to a solid salt by treating it with a suitable acid. Due to the instability of the dioxolanic compounds in an aqueous acid medium it is preferable to use for its salification weak acids such as maleic, citric, orotic, acetic, fumaric, oxalic, malonic or p-toluenesulphonic acid, in any adequate organic solvent such as acetone, methanol, ethanol, isopropanol, chloroform- ethyl acetate, benzene and in general any solvent which would adequately dissolve the amine.

When the acid used has at the same time geometric or optical isomerism, in the formation of the corresponding salt, one of the two diastereoisomers may be preferably separated by crystallization. Such is the case with maleate of which we detail the preparation below by way of example, with the analytical physical data of the other salts as well.

Example 2

Preparation of the maleate of the ITA 362.

To a solution of 166 g (0,43 m) of ITA 362 in 400 cc of acetone at 40—50 deg.C is added 50 g (0,434 m) of maleic acid dissolved in acetone at the same temperature. The mixture is stirred and immediately a white solid starts separating out until it forms a compact mass. Leave it to cool, filter it, wash it with acetone and vacuum dry on $Cl_2Ca$, obtaining 134 g (62%) of a white crystalline solid. Melting point: (m.p.) 163—164 deg.C.

Potentiometric evaluation of the acid groups: 100,0%.

Potentiometric evaluation of the basic groups: 101,0%.

IR (KBr): characteristic bands at 1580, 1480—1450, 1360, 1090, 1020—970 and 760 cm$^{-1}$.

NMR ($d_6$-DMSO): The spectrum with minor variations in the displacement and in the form of the signals is very similar to that of ITA 362 base except for a new signal at 6,3 ppm corresponding to the olefin protons of the maleic acid and the signals of the furan proton which appear as two singlets at 6,70 and 6,55 ppm in the free base. While in the base both signals have a similar intensity, with only the most deshielded being slightly more intense, (10—20%) in the salt this signal clearly predominates with respect to the other in a proportion of 3:1.

Concentrating the mother liquor until almost dryness and crystallizing anew with ethyl acetate one obtains 38 g (18%) of a white crystalline solid. m.p.: 143—143,5 deg.C.

Potentiometric evaluation of the acid groups: 100,8%.

Potentiometric evaluation of the basic groups: 99,1%.

IR (KBr): A spectrum very similar to that of the first fraction with minor variations, though significant, in the form and the intensity of the bands.

NMR ($d_6$-DMSO): A spectrum practically identical to the first fraction except for the signals corresponding to the furan proton. In this case the signal at 6,55 ppm undoubtedly predominates, in the proportion of 4:1.

Example 3

Preparation of orotate of the ITA 362

On heating with reflux in ethanol a stechiometric mixture of the ITA 362 base and orotic acid, a white crystalline solid separates out in parallel with the transformation of the acid that previously has not completely dissolved.

m.p.: 235—6 deg. C (d).

IR (KBr): characteristic bands at 1710, 1630, 1410, 1100 and 755 $cm^{-1}$.

Working in a manner generally similar to that indicated above, and in accordance with well established techniques one obtains the citrate, oxalate, malonate and the p-toluenesulphonate of the ITA 362 and for which the analytical data is given in the following examples.

Example 4

Preparation of the citrate of the ITA 362.

The citrate is a yellowish highly hygroscopic product.

m.p.: commences to soften at 52 deg.C, decomposing at 90°C.

Potentiometric evaluation of the basic groups: 103,4%.

Example 5

Preparation of the oxalate of the ITA 362.

m.p.: 178—180 deg.C.

IR (KBr): characteristic bands at 1720 to 1700, 1475, 1455, 1255, 1165 and 1090 $cm^{-1}$.

Example 6

Preparation of the malonate of the ITA 362.

m.p.: 111—114 deg.C.

IR (KBr): characteristic bands at 1720—1600, 1490, 1455, 1090, 830 and 760 $cm^{-1}$.

Example 7

Preparation of the p-toluenesulphonate of the ITA 362

m.p.: 99—103 deg.C.

IR (KBr): characteristic bands at 3390, 1490, 1455, 1190, 1040, 1020, 820, 760 and 690 $cm^{-1}$.

PHARMACOLOGICAL ACTIVITY

*Acute toxicity*

The acute toxicity of the ITA 362 with both oral and intra peritoneal administration in the mouse and the rat has been studied using as reference the Amiodarona (The Merck Index, 9th edition,. Merck Co. page 498, 1976) administered under the same conditions.

The respective lethal dose 50 was calculated according to the method of Litchfield and Wilcoxon (Jl. Pharmacol. Exptl. 96, 99, 1949). The values obtained are shown in the tables 1, and 2 given below.

TABLE 1

Acute toxicity of ITA 362 in the rat and in the mouse Oral administration.

| Treatment | Species | $LD_{50}$ in mg/kg |
|---|---|---|
| ITA 362 maleate | Rat | > 4000 |
| ITA 362 maleate | Mouse | > 4000 |
| Amiodarona | Mouse | > 4000 |

TABLE 2
Acute toxicity of ITA 362 in the rat and in the mouse Intraperitoneal administration.

| Treatment | Species | $LD_{50}$ and confidence limits in mg/kg. |
|---|---|---|
| ITA 362 maleate | Rat | 105 (83—133) |
| Amiodarona | Rat | 885 (776—1010) |
| ITA 362 maleate | Mouse | 106 (102—110) |
| Amiodarona | Mouse | 940 (813—1086) |

*Vasodilatory, antiangina and anti-arrythmic activity*

In the table 3 is shown the results of the vasodilatory activity of the ITA 362 in the perfusion test of the hindquarters of rats with a hyperpotassemic solution. (F. N. Fastier, F. M. Smirk. Jl. Pharmacolog. Exp. Therapy. 89. 256, 1947).

The activity turned out to be similar to that of the nitroglycerine which was used as the reference thus demonstrating clearly the power of the compound.

The antiangina activity was also compared with that of the nitroglycerine by means of the amplitude increase protection test of the T wave for vasopression in the conscious dog. (A. Lindner, M. London, G. Werner, Scheiz. Med. Wsohr. *83*, 360, 1953; J. Papp, L. Szekeres. Arch. Int. Pharmacodyn *160*, 1, 1966). The dose of 1 mg/kg with intravenous administration of the ITA 362 shows almost a 50% protection, its activity being greater than that of nitroglycerine which was used as the reference at a dose of 0,4 mg/kg. The results are shown in the table 4.

Tables 5 and 6 show the anti-arrithmic activity of the ITA 362 in the male NMRI mouse by oral and intraperitoneal route using the fibrillation test by chloroform in the mouse. (V. W. Lawson, J. Pharmacol. Exp. Ther. *160*, 22, 1968; B. Vargaftig, J. L. Coignet. Eur. J. Pharmacol. *6*, 49, 1969).

In this test Amiodarona was used as the reference and was compared with the activities of the various salts of the ITA 362. The activity of the two forms of maleate described in the example 2 were studied and which in the table are subtitled as isomer A corresponding to the first fraction obtained in the aforementioned example, and isomer B corresponding to the second fraction.

The calculated $ED_{50}$ are similar there being no significant statistical difference between either the two isomers or in relation to the citrate. Table 6 illustrates that the activity is comparable to that of the Amiodarona.

TABLE 3
*Vasodilatory activity.* Perfusion of the hind quarters of rats with a hyperpotassemic solution.

| Treatment | Concentration in g/ml | No. of animals | % drop in pressure | $ED_{25}$ mg/kg |
|---|---|---|---|---|
| Nitroglycerine | $10^{-5}$ | 10 | 25,4 | |
| | $10^{-6}$ | 16 | 24,7 | |
| | $10^{-7}$ | 6 | 17,5 | $4,6 . 10^{-6}$ |
| | $10^{-8}$ | 7 | 10,9 | r = 0,9590 |
| ITA 362 | $10^{-5}$ | 7 | 33,6 | |
| citrate | $10^{-6}$ | 20 | 16,3 | $2,61 . 10^{-6}$ |
| | $10^{-7}$ | 15 | 12,7 | r = 0,8605 |
| | $10^{-8}$ | 6 | 12,5 | |

TABLE 4

*Anti-angina activity.* Amplitude increase protection of the T wave by vasopression in the conscious dog.

| Treatment | Weight kg. | Dose i.v. mg/kg | Control quot. treatment | t student | % Prot. |
|---|---|---|---|---|---|
| ITA 362 maleate | 14,0 ± 1,2 | 1 | 1,893 | p<0,025 | 46,8 |
| Nitroglycerine at 1% | 15,3 ±1 1,2 | 0,4 | 1,520 | n.s. | 28,9 |

TABLE 5

*Anti-arrythmic activity.* Fibrillation by chloroform in the male NMRI mouse. Route: p.o.

| Treatment | Dose. p.o. mg/kg | No. of animals | % protection versus fibrillation | $ED_{50}$ and confidence limits p<0,05 |
|---|---|---|---|---|
| Control | 25 ml/kg | 20 | 15 | ——— |
| ITA 362 citrate | 100 | 20 | 55 | 43,21 (*) |
| | 50 | 20 | 55 | |
| | 25 | 20 | 45 | |
| | 12,5 | 19 | 31,6 | |
| | 6,25 | 19 | 42,1 | |
| | 3,125 | 20 | 10 | |
| ITA 362 maleate (isomer A) | 100 | 20 | 70 | 35,4 (27,52 45,42) |
| | 50 | 20 | 70 | |
| | 25 | 20 | 35 | |
| | 12,5 | 20 | 25 | |
| ITA 362 maleate (isomer B) | 100 | 20 | 85 | 27,60 (16,39 45,48) |
| | 50 | 20 | 45 | |
| | 25 | 20 | 55 | |
| | 12,5 | 20 | 35 | |

* calculation is not possible

TABLE 6

*Anti-arrythmic activity.* Fibrillation by chloroform in the male NMRI mouse. Route: i.p.

| Treatment | Dose. i.p. mg/kg | No. of animals | % protection versus fibrillation | $ED_{50}$ and confidence limits p<0,05 |
|---|---|---|---|---|
| Control | 25 ml/kg | 20 | 5 | ———— |
| Amiodarona | 100 | 20 | 89,5 | 34,0 |
| | 50 | 20 | 52,6 | (21,4 |
| | 25 | 20 | 45 | 54,0) |
| ITA 362 citrate | 100 | 20 | 84,2 | |
| | 50 | 20 | 35 | 41,4 |
| | 25 | 20 | 40 | (27,1 |
| | 12,5 | 20 | 25 | 63,2) |
| ITA 362 maleate (isomer A) | 100 | 20 | 70 | |
| | 50 | 20 | 60 | 35,3 |
| | 25 | 20 | 35 | (20.17 |
| | 12,5 | 20 | 35 | 61,98) |
| ITA 362 maleate (isomer B) | 100 | 20 | 95 | |
| | 50 | 20 | 70 | 24,5 |
| | 25 | 20 | 35 | (16,11 |
| | 12,5 | 20 | 40 | 37,20) |

*Dosage*

The daily dose of the active pharmaceutical product may vary over a wide margin between 5 and 1000 mg depending on the therapeutic application and the form of administration.

*Forms of Administration*

The new product ITA 362 described above and obtained by means of the method of preparation object of this present invention may be administered under the various conventional forms as for example by tablets, dragees, capsules or in suspension with a view to administration by oral or by intravenous injection.

The pharmaceutical specialty contains the active compound and one or other of the acceptable pharmaceutical vehicles as well as auxiliary non-toxic substances such as dispersal agents, compacting agents, emulsifiers, preservatives, humectants or others with particularly desirable specific properties.

As non-limitative illustrative examples of suitable pharmaceutical specialties containing the active product prepared according to the method object of this invention are the following.

Example 8

*Capsules.* Containing 200 mg of active ingredient.

1 Capsule No. 1

| | |
|---|---|
| 2-(2'-benzofuryl)-4-butylaminomethyl--2-p-chlorophenyl-1,3-dioxolane, maleate | 200 mg |
| Lactose USP (USP = United States Pharmacopea) | 45 mg |
| Magnesium stearate | 5 mg |
| | 250 mg |

Example 9

*Tablets*

Tablets with 25 mg of active ingredient

| | |
|---|---|
| 2-(2'-benzofuryl)-4-butylaminomethyl -2-p-chlorophenyl-1,3-dioxolane, maleate | 25 mg |
| Starch USP | 2,5 mg |
| Talc USP | 2 mg |
| Magnesium stearate | 0,5 mg |
| | 30 mg |

*Therapeutic Applications*

The use of this compound is recommended for the treatment of angina pectoris, stenocardia and acute visceral pain. Prophylaxis of the effort, stress, and nocturnal angor. Treatment in the recuperation stage of miocardiac infarct. Ventricular insufficiency of left side congestion. .Pulmonary oedema. Heart surgery. Anaesthesia. Supra-ventricular and ventricular arrythmia or arrythmia resistant to the classic treatments, Sinus tachycardia and paroxysmal tachycardia, auricular and flutter fibrillation, nodal and ventricular tachycardia, ventricular and auricular extrasystoles and the Wolf-Parkinson-White syndrome.

**Claims**

1. A dioxolanic amine with pharmacological activity of the structural formula I

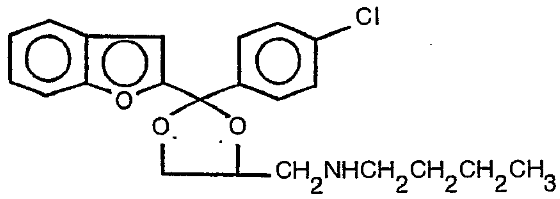

I

corresponding to the 2-(2'-benzofuryl)-4-n-butylaminomethyl-2-p-chlorophenyl-1,3-dioxolane, as well as its pharmaceutically acceptable salts.

2. A method of preparation of the dioxolanic amine with pharmacological activity of the general formula I as has been defined in Claim 1, characterized by starting from a bromomethyl dioxolanic derivative of the structural formula II:

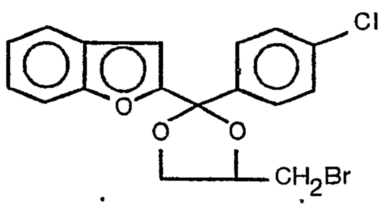

II

corresponding to the 2-(2'-benzofuryl)-4-bromomethyl-2-p-chlorophenyl-1,3-dioxolane which is treated with n-butylamine in an inert anhydrous solvent, preferably an aromatic hydrocarbon and in the presence of an acid acceptor at the boiling point temperature of the mixture during a period varying from between 5 to 50 hours after which the mixture is allowed to cool; a solution of sodium or potassium hydroxide is added; the organic phase is then decanted, washed and dried, and the solvent removed, product I being obtained thus, in the form of an oil which can subsequently be purified by distillation at a reduced pressure or by the formation of solid salts by treating with an acid in a suitable solvent.

3. The method as in claim 2, characterized in that the acid acceptor utilized in the reaction for the preparation is the n-butylamine itself, it being required to use at least two mols. of this for each mol. of bromomethyl derivative II.

4. The method as in claim 2, characterized in that the acid acceptor utilized in the preparation reaction is a tertiary amine.

5. The method as in claim 2, characterized in that the acid acceptor utilized in the preparation reaction is an inorganic base.

6. The method according to any of claims 2 to 5, characterized in that the final compound I is purified by means of the formation of salts treating the base with an acid, preferably a weak organic acid, in a suitable organic solvent; then separating off the solid salt which is formed, by filtration.

7. The method according to claim 6, characterized in that the acid used has at the same time geometric and optical isomerism which on the formation of the corresponding salts, the two possible diastereoisomers are separated by fractional crystallization obtaining in this manner the resolution of the isomers.

8. A dioxolanic compound of the following structural formula II

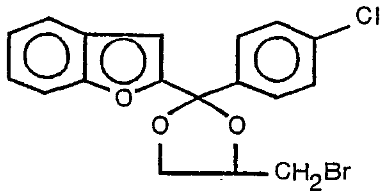

II

corresponding to the 2-(2′-benzofuryl)-4-bromomethyl-2-p-chlorophenyl-1,3-dioxolane.

9. A method for the preparation of a dioxolanic compound of structural formula II, as has been defined in claim 8, characterized by starting from the ketone of formula III

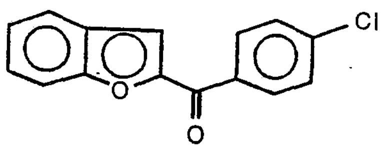

III

dissolved in a chlorinated solvent such as methylene chloride, chloroform or carbon tetrachloride and at a low temperature, preferably between 0 and 5°C with a quantity of between one and three times mol. with respect to the ketone III of epibromhydrin and a Lewis acid as catalyst, then completing the reaction at a temperature of between ambient temperature and the boiling point of the mixture during a period varying between 2 and 24 hours, then separating the new bromomethyl dioxolanic derivative of formula II.

10. The method as in claim 9, characterized in that the Lewis acid utilized as a catalyst is tin tetrachloride in a quantity of the order of one tenth part mol with respect to the epibromhydrin.

11. The method according to claim 9, characterized in that the Lewis acid utilized as a catalyst is boron trifluoride in a quantity of the order of one tenth part mol with respect to the epibromhydrin.

12. The method as in claim 9, characterized in that the Lewis acid utilized as a catalyst is anhydrous aluminium trichloride in a quantity of the order of one tenth part mol. with respect to the epibromhydrin.

13. A pharmaceutical composition characterized by its containing as active ingredient the dioxolanic amine with pharmacological activity of general formula I, defined in the claim 1.

14. A pharmaceutical composition according to claim 13 for use in the treatment of angina pectoris, stenocardia and acute visceral pain, prophylaxis of the effort, stress, and nocturnal angor, treatment in the recuperation stage or miocardiac infarct, treatment in the cases of cogestive insufficiency of the left ventricular, pulmonary oedema, heart surgery, anaesthesia supra ventricular and ventricular arrythmai, arrythmia resistant to the classic treatments, sinus and proxysmal tachycardia, auricular and flutter fibrillation, nodal and ventricular tachycardia, ventricular and auricular extrasystoles and the Wolf-Parkinson-White syndrome.

**Patentansprüche**

1. Dioxolanisches Amin mit pharmakologischer Aktivität und der Strukturformel I

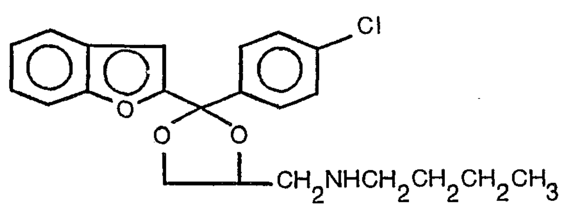

I

entsprechend einem 2-(2′-Benzofuryl)-4-n-butylaminomethyl-2-p-chlorphenyl-1,3-dioxolan und die pharmazeutisch verträglichen Salze.

2. Verfahren zur Herstellung des dioxolanischen Amins mit pharmakologischer Aktivität und der allgemeinen Formel I nach Anspruch 1, dadurch gekennzeichnet, daß man, ausgehend von einem Brommethyl-Derivat der dioxolanischen Verbindung mit der Strukturformel II,

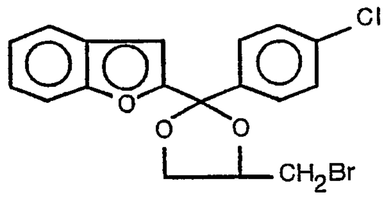

II

entsprechend einem 2-(2'-Benzofuryl)-4-brommethyl-2-p-chlorphenyl-1,3-dioxolan, dieses mit n-Butylamin in einem inerten, wasserfreien Lösungsmittel, vorzugsweise in einem aromatischen Kohlenwasserstoff und in Gegenwart eines Säure-Acceptors bei der Siedepunkttemperatur der Mischung für einen Zeitraum von zwischen 5—50 Stunden behandelt und anschließend die Mischung abkühlen laßt; eine Natrium- oder Kaliumhydroxid-Lösung zugibt, die organische Phase anschließend abdekantiert, wäscht und trocknet, das Lösungsmittel entfernt, und do das Produkt I in Form eines Öls erhält, das anschließend durch Destillation bei vermindertem Druck oder durch Bildung von festen Salzen durch Behandlung mit einer Säure in einem geeigneten Lösungsmittel, gereinigt werden kann.

3. Verfahren nach Anspruch 2, dadurch gekennzeichnet, daß der in der Herstellungsreaktion verwendete Säure-Acceptor n-Butylamin selbst ist, wobei es erforderlich ist, zumindest 2 Mol für jedes Mol des Brommethylderivates II zu verwenden.

4. Verfahren nach Anspruch 2, dadurch gekennzeichnet, daß der in der Herstellungsreaktion verwendete Säure-Acceptor ein tertiäres Amin ist.

5. Verfahren nach Anspruch 2, dadurch gekennzeichnet, daß der in der Herstellungsreaktion verwendete Säure-Acceptor eine anorganische Base ist.

6. Verfahren nach Anspruch 2 bis 5, dadurch gekennzeichnet, daß man das Endprodukt I mittels Salzbildung durch Behandlung der Base mit einer Säure, vorzugsweise einer schwachen organischen Säure in einem geeigneten organischen Lösungsmittel reinigt und anschließend das gebildete feste Salz durch Filtration abtrennt.

7. Verfahren nach Anspruch 6, dadurch gekennzeichnet, daß die verwendete Säure gleichzeitig sowohl eine geometrische als auch optische Isomerie aufweist, wobei durch die Bildung der entsprechenden Salze die zwei möglichen Diastereoisomeren durch fraktionierte Kristallisation abgetrennt werden und man auf diese Weise die Auftrennung der Isomeren erreicht.

8. Dioxolanische Verbindung, dadurch gekennzeichnet, daß sie die Strukturformel II aufweist

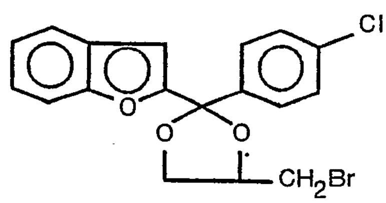

II

entsprechend einem 2-(2'-Benzofuryl)-4-brommethyl-2-p-chlorphenyl-1,3-dioxolan.

9. Verfahren zur Herstellung einer dioxolanischen Verbindung der Strukturformel II nach Anspruch 8, dadurch gekennzeichnet, daß man ausgehend von einem Keton der Formel III,

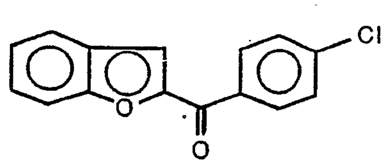

III

das in einem chlorierten Lösungsmittel, beispielsweise Methylenchlorid, Chloroform oder Kohlenstofftetrachlorid gelöst ist, dieses bei niedriger Temperatur, vorzugsweise zwischen 0 und 5°C mit einer Menge zwischen 1 und 3 Mol, bezogen auf das Keton III Epibromhydrin und einer Lewis-Säure als Katalysator umsetzt, dann die Reaktion bei einer Temperatur zwischen Raumtemperatur und dem Siedepunkt der Mischung für einen Zeitraum zwischen 2 und 24 Stunden zum Abschluß bringt und anschließend das neue Brommethyl-Dioxolan-Derivat der Formel II abtrennt.

10. Verfahren nach Anspruch 9, dadurch gekennzeichnet, daß die als Katalysator verwendete Lewis-Säure Zinntetrachlorid in einer Menge im Bereich von einem Zehntel Molteil, bezogen auf das Epibromhydrin, ist.

11. Verfahren nach Anspruch 9, dadurch gekennzeichnet, daß die als Katalysator verwendete Lewis-Säure Bortrifluorid in einer Menge in der Größenordnung von einem Zehntel Molteil bezogen auf das Epibromhydrin ist.

12. Verfahren nach Anspruch 9, dadurch gekennzeichnet, daß die als Katalysator verwendete Lewis-Säure wasserfreies Aluminiumtrichlorid in einer Menge der Größenordnung von einem Zehntel Molteil bezogen auf das Epibromhydrin ist.

13. Pharmazeutische Zubereitung, dadurch gekennzeichnet, daß sie als aktiven Bestandteil das dioxolanische Amin mit pharmakologischer Aktivität gemäß der allgemeinen Formel I nach Anspruch 1, enthält.

14. Pharmazeutische Zubereitung nach Anspruch 13, dadurch gekennzeichnet, daß man sie zur Behandlung von Angina pectoris, der Stenokardie und akuter visceraler Schmerzen, in der Prophylaxe-Behandlung von Angst-, Stress- und Nacht-Angstzuständen, zur Behandlung in der Erholungsphase des Myokardinfarktes, zur Behandlung in Fällen der angeborenen Insuffizienz des linken Ventrikels, zur Behandlung des Lungenödems, bei Herzoperationen, in der supraventrikularen Anästhesie und zur Behandlung von ventrikularen Arrythmien sowie zur Behandlung von Arrythmien, die gegen die klassische Behandlung resistent sind, zur Behandlung der Sinus- und proximalen Tachykardie, von aurikulärem und Flimmer-Flattern, zur Behandlung nodaler und ventrikulärer Tachykardie, von ventrikulären und aurikulären Extrasystolen und zur Behandlung des Wolf-Parkinson-White-Syndroms, verwendet.

**Revendications**

1. Amine dioxolanique ayant de l'activité pharmacologique, de formule de structure I

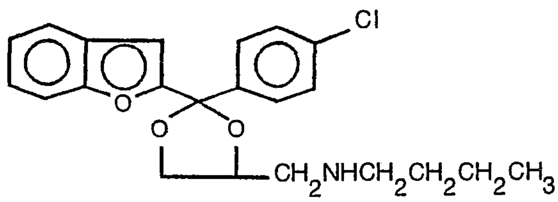

I

correspondant au 2-(2'-benzofuryl)-4-n-butylaminométhyl-2-p-chlorophényl-1,3-dioxolane, de même que ses sels pharmaceutiquement acceptables.

2. Procédé de préparation de l'amine dioxolanique à activité pharmacologique de formule générale I telle que définie à la revendication 1, caractérisé en ce qu'on part d'un dérivé dioxolanique bromométhylé de formule structurale II:

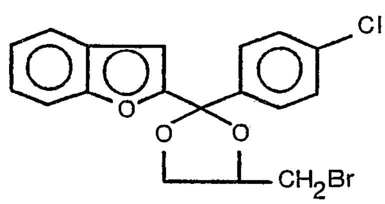

II

correspondant au 2-(2'-benzofuryl)-4-bromométhyl-2-p-chlorophényl-1,3-dioxolane qui est traité avec de la n-butylamine dans un solvant anhydre inerte, de préférence un hydrocarbure aromatique, et en la présence d'un accepteur d'acide à la température du point d'ébullition du mélange au cours d'une période variant entre 5 et 50 heures, après quoi on laisse refroidir le mélange, on ajoute une solution d'hydroxyde de sodium ou de potassium, on décante ensuite la phase organique, on la lave et la sèche et l'on élimine le solvant, en obtenant ainsi le produit I sous la forme d'une huile qui peut être purifiée par la suite par distillation sous pression réduite ou par formation de sels solides par traitement avec un acide dans un solvant convenable.

3. Procédé selon la revendication 2, caractérisé en ce que l'accepteur d'acide utilisé dans la réaction pour la préparation est la n-butylamine elle-même, étant requis d'utiliser au moins deux moles de celle-ci pour chaque mole du dérivé bromométhylé II.

4. Procédé selon la revendication 2, caractérisé en ce que l'accepteur d'acide utilisé dans la réaction de préparation est une amine tertiaire.

5. Procédé selon la revendication 2, caractérisé en ce que l'accepteur d'acide utilisé dans la réaction de préparation est une base minérale.

6. Procédé selon l'une quelconque des revendications 2 à 5, caractérisé en ce que le composé final I est purifié au moyen de la formation de sels par traitement de la base avec un acide, de préférence un acide organique faible, dans un solvant organique convenable, puis en ce qu'on sépare le sel solide qui est formé par filtration.

7. Procédé selon la revendication 6, caractérisé en ce que l'acide utilisé a en même temps l'isomérisme géométrique et optique qui correspond à la formation des sels correspondants, en ce qu'on sépare les deux diastéréoisomères possibles par cristallation fractionnée, en obtenant ainsi la résolution des isomères.

8. Composé dioxolanique de formule structurale II suivante:

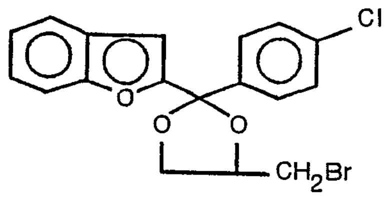

II

correspondant au 2-(2'-benzofuryl)-4-bromométhyl-2-p-chlorophényl-1,3-dioxolane.

9. Procédé de préparation d'un composé dioxolanique de formule de structure II tel que défini à la revendication 8, caractérisé en ce qu'on part de la cétone de formule III:

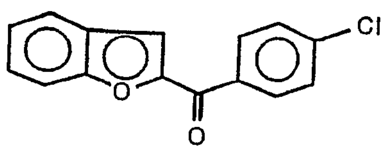

III

dissoute dans un solvant chloré comme le chlorure de méthylène, le chloroforme ou le tétrachlorure de carbone, à une température basse, de préférence entre 0 et 5°C, on la fait réagir avec une quantité entre une et trois fois molaire, par rapport à la cétone III, d'épibromhydrine et un acide de Lewis comme catalyseur, en complétant alors la réaction à une température entre la température ambiante et le point d'ébullition du mélange pendant une période variant entre 2 et 24 heures, puis en ce qu'on sépare le nouveau dérivé dioxolanique bromométhylé de formule II.

10. Procédé selon la revendication 9, caractérisé en ce que l'acide de Lewis utilisé comme catalyseur est du tétrachlorure d'étain en une quantité de l'ordre de un dixième de partie en mole par rapport à l'épibromhydrine.

11. Procédé selon la revendication 9, caractérisé en ce que l'acide de Lewis utilisé comme catalyseur est du trifluorure de bore en une quantité de l'ordre de un dixième de partie en mole par rapport à l'épibromhydrine.

12. Procédé selon la revendication 9, caractérisé en ce que l'acide de Lewis utilisé comme catalyseur est un trichlorure d'aluminium anhydre en une quantité de l'ordre de un dixième de partie en mole par rapport à l'épibromhydrine.

13. Composition pharmaceutique, caractérisée par sa teneur en tant qu'ingrédient actif de l'amine dioxolanique à activité pharmacologique de formule générale I définie à la revendication 1.

14. Composition pharmaceutique selon la revendication 13 pour l'utilisation dans le traitement de l'angine de poitrine, de la sténocardie et de la douleur viscérale aiguë, la prophylaxie de l'effort, le stress et l'angoisse nocturne, le traitement dans le stade de récupération de l'infarctus du miocarde, le traitement dans les cas d'insuffisance congestive du ventricule gauche, l'oedème pulmonaire, la chirurgie du coeur, l'anesthésie supraventriculaire et l'arythmie ventriculaire, l'arythmie résistant aux traitements classiques, la tachycardie sinusmale et paroxysmale, la fibrillation auriculaire et de palpitation, la tachycardie nodale et ventriculaire, les extrasystoles ventriculaires et auriculaires et le syndrome de Wolf-Parkinson-White.